(19) Europäisches Patentamt
European Patent Office
Office européen des brevets

(11) Numéro de publication: **0 055 198**
**B1**

# (12) FASCICULE DE BREVET EUROPEEN

(45) Date de publication du fascicule du brevet: **04.04.84**

(51) Int. Cl.³: **C 07 C 37/00**, C 07 C 39/27

(21) Numéro de dépôt: **81420187.7**

(22) Date de dépôt: **18.12.81**

(54) **Procédé de préparation de phénols métachlorés.**

(30) Priorité: **24.12.80 FR 8027938**

(43) Date de publication de la demande: **30.06.82 Bulletin 82/26**

(45) Mention de la délivrance du brevet: **04.04.84 Bulletin 84/14**

(84) Etats contractants désignés: **BE DE FR GB IT LU NL**

(56) Documents cités:
**EP - A - 0 008 061**
**DE - A - 2 331 712**
**FR - A - 2 209 738**
**FR - A - 2 285 355**
**US - A - 2 725 402**

**CHEMICAL ABSTRACTS, vol. 86, no. 21, 23 mai 1977, réf. no. 155305c page 440 COLUMBUS OHIO (US)**

(73) Titulaire: **RHONE-POULENC SPECIALITES CHIMIQUES, "Les Miroirs" 18, Avenue d'Alsace, F-92400 Courbevoie (FR)**

(72) Inventeur: **Cordier, Georges, 50, chemin des Hermières, F-69340 - Francheville (FR)**

(74) Mandataire: **Vignally, Noel et al, RHONE-POULENC RECHERCHES Centre de recherches de Saint Fons Service Brevets Boîte postale 62, F-69190 Saint Fons (FR)**

**EP 0 055 198 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Procédé de préparation de phénols métachlorés

La présente invention a pour objet un procédé de préparation de phénols comportant un atome de chlore sur l'une au moins des positions méta par rapport à l'hydroxyle phénolique, par hydrodéchloration de chlorophénols plus fortement chlorés.

Par la suite, pour des raisons de commodité, on désignera par l'expression »phénols métachlorés« les phénols comportant un atome de chlore sur l'une au moins des positions méta.

Les phénols métachlorés, et en particulier le chloro-3 phénol et le dichloro-3,5 phénol, sont des composés qui revêtent un très grand intérêt industriel comme intermédiaires en synthèse organique.

Diverses méthodes de préparation des phénols métachlorés ont été proposées. On distingue notamment des méthodes engendrant la fonction phénol sur des composés aromatiques chlorés (par exemple l'hydrolyse alcaline des polychlorobenzènes; la nitration des chloro-3 et dichloro-3,5 benzènes suivie de la réduction du groupe nitro en groupe amino, de la diazotation de ce dernier et de la décomposition du sel de diazonium); des méthodes de chloration de phénols et des méthodes de déchloration de polychlorophénols. Cette dernière méthode présente un très grand intérêt industriel en raison de la disponibilité des polychlorophénols dont certains sont des produits courants et d'autres des sous-produits d'intérêt limité qu'il importe de valoriser.

C'est ainsi, par exemple, que l'on obtient des tri- et tétrachlorophénols isomères dont certains comportent on ou deux atomes de chlore en position méta par rapport à l'hydroxyle phénolique lors de la préparation du tétrachloro-2,3,4,6 phénol et du pentachlorophénol par chloration du dichloro-2,6 phénol sous-produit de la préparation du dichloro-2,4 phénol. Ces divers polychlorophénols constituent des matières de choix pour la préparation des phénols métachlorés par déchloration. Une méthode d'élimination des atomes de chlore excédentaires consiste à soumettre les polychlorophénols à une hydrogénation en phase vapeur ou en phase liquide en présence d'un catalyseur. Pour des raisons de simplification, on désignera par la suite par »hydrodéchloration« la déchloration des polychlorophénols par hydrogénation.

Le problème essentiel posé par l'hydrodéchloration des polychlorophénols en chloro-3 ou dichloro-3,5 phénols réside dans l'élimination sélective des atomes de chlore en position 2 et/ou 4 et/ou 6 par rapport à l'hydroxyle phénolique. On a proposé divers procédés d'hydrodéchloration des polychlorophénols mais aucun ne s'est révélé jusqu'ici pleinement satisfaisant.

Ainsi, dans le brevet américain 2 803 669 délivré le 20 août 1957 on a décrit un procédé d'hydrodéchloration de polychlorophénols en phase vapeur par passage d'un mélange gazeux hydrogène/polychlorophénols sur un catalyseur à base d'halogénures cuivreux (chlorure cuivreux par exemple) déposés sur de l'alumine, maintenu à température élevée (350 à 550°C). Appliqué à l'hydrodéchloration du tétrachloro-2,3,4,6 phénol, ce procédé n'a pas permis d'obtenir une élimination sélective des atomes de chlore en position 2, 4 et 6 par rapport à l'hydroxyle phénolique. La masse réactionnelle résultant de l'hydrogénation est constituée pour l'essentiel de dichloro-2,4 et de dichloro-2,6 phénols.

Dans la demande de brevet français No. 73-43.484 publiée sous le No. 2 209 738, on a proposé un procédé de préparation de phénols métahalogénés par déshalogénation des polyhalogénophénols par hydrogénation en phase liquide à température élevée, en présence d'un catalyseur comportant soit un ou plusieurs sulfures ou polysulfures de fer, de nickel ou de cobalt, soit un métal noble tel que le palladium ou le platine associé à un dérivé du soufre. La réaction est de préférence conduite en présence d'une base telle que les hydroxydes ou les carbonates alcalins pour neutraliser les hydracides engendrés par la réaction au fur et à mesure de leur formation. Bien que ce procédé se montre très sélectif vis-à-vis de la formation des phénols métachlorés, il présente l'inconvénient de devoir être mis en oeuvre en présence d'une base et en particulier d'une base alcaline dans des conditions de température (cette dernière doit être comprise de préférence entre 180 et 330°C) favorables à la formation d'halogénodioxines et en particulier de polychlorodioxines dont on sait que certaines présentent une toxicité élevée. Un tel inconvénient enlève en pratique tout intérêt industriel à ce procédé. En définitive l'industrie est encore à la recherche d'un procédé sélectif d'obtention de phénols métachlorés par hydrodéchloration de polychlorophénols s'affranchissant de la présence de bases alcalines. La présente invention se propose précisément de mettre à la disposition de l'industrie un procédé d'hydrodéchloration sélective des polychlorophénols exempt des inconvénients précités.

Plus spécifiquement, la présente invention a pour objet un procédé d'obtention sélective de chlorophénols comportant un atome de chlore sur l'une au moins des positions méta par rapport à l'hydroxyle phénolique, par hydrogénation à chaud en phase liquide, en présence d'un catalyseur à base d'un métal noble du groupe VIII de la classification périodique, de polychlorophénols de formule générale (I):

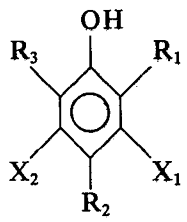

(I)

dans laquelle:

— $X_1$ et $X_2$, identiques ou différents représentent un atome de chlore, un atome d'hydrogène, un radical alkyle, aryle, arylalkyle, alkyloxy, aryloxy, l'un au moins des symboles $X_1$ et $X_2$ représentant un atome de chlore,

— $R_1$, $R_2$ et $R_3$, identiques ou différents, représentent un atome de chlore, un atome d'hydrogène, un radical alkyle, un radical aryle, arylalkyle, un radical alkyloxy, aryloxy, l'un au moins des symboles $R_1$, $R_2$ et $R_3$ représentant un atome de chlore,

caractérisé en ce que la réaction est conduite en phase organique en présence d'un acide de Lewis.

Dans la formule (I), ceux des radicaux $X_1$, $X_2$, $R_1$ et $R_3$ qui ne symbolisent pas un atome de chlore représentent plus spécifiquement un radical alkyle comportant de 1 à 10 atomes de carbone et de préférence de 1 à 4 atomes de carbone comme les radicaux méthyle, éthyle, n-propyle, isopropyle, n-butyle, isobutyle, t-butyle; un radical phényle; un radical benzyle; un radical alkyloxy comportant de 1 à 10, et de préférence de 1 à 4 atomes de carbone comme les radicaux méthoxy, éthoxy, n-propyloxy, isopropyloxy, n-butyloxy; le radical phénoxy.

Par acide de Lewis on désigne, conformément à la définition usuelle, des composés accepteurs de doublets électroniques. Pour la mise en œuvre de la présente invention, on pourra faire appel à tous les types d'acides de Lewis et notamment à ceux cités dans l'ouvrage édité par G. A. OLAH »Friedel-Crafts and Related Reactions« 1963 tome I, pages 191 à 197. Parmi les acides de Lewis, on utilise de préférence les halogénures acides, cf.: G. A. OLAH loc. cit. pages 215 à 219 et plus particulièrement, les halogénures d'éléments des groupes 3a, 4a, 5a, 1b, 2b, 4b, 5b, 6b, 7b et 8 de la classification périodique des éléments (cf. Handbook of Chemistry and Physics édité par R. C. WEAST 53ème édition 1972—1973) tels que les chlorures, bromures, fluorures et iodures de bore, d'aluminium, d'étain, de phosphore, d'arsenic, de bismuth, de titane, de zirconium, de vanadium, de molybdène, de fer, de cobalt, de nickel, de cuivre, de zinc et de cadmium. Comme exemples spécifiques de tels halogénures on peut citer: le trichlorure d'aluminium, le tribromure d'aluminium, le triiodure d'aluminium, les chlorures stannique et stanneux, les bromures stannique et stanneux, le trichlorure de bismuth, le tribromure de bismuth, le trifluorure de bore et ses complexes avec des composés électrodonneurs tels que les éthers (par exemple le diéthyltrifluoroéthérate de bore), le trichlorure de bore, le tribromure de bore, les tétrachlorures de titane, de zirconium et de vanadium, les chlorures de molybdène, le chlorure ferrique, le bromure ferrique, le chlorure cuivreux, le chlorure cuivrique, le chlorure de zinc. Parmi les halogénures cités précédemment, on met en œuvre de préférence le trichlorure et le tribromure d'aluminium.

Il est bien clair que l'on peut indifféremment utiliser un seul acide de Lewis ou un mélange de plusieurs acides de Lewis dans la mise en œuvre du procédé selon l'invention.

La quantité d'acide de Lewis exprimée par le rapport molaire acide de Lewis/polychlorophénol peut varier dans de larges limites. De préférence la quantité d'acide de Lewis est calculée pour le que rapport molaire précité soit d'au moins $1 \times 10^{-4}$ et de préférence $1 \times 10^{-2}$. Il n'y a pas de limite supérieure critique de ce rapport mais pour des raisons pratiques évidentes, il n'y a pas lieu qu'il soit supérieur à 2 et de préférence supérieur à 1.

Le milieu réactionnel peut être constitué par le ou les polychlorophénol(s) soumis à l'hydrodéchloration s'ils sont liquides dans les conditions de la réaction ou par un ou plusieurs solvants inertes dans lesdites conditions. Comme exemples de solvants, on peut citer: des hydrocarbures aliphatiques tels que l'octane, l'hexane; des hydrocarbures cycloaliphatiques tels que le cyclohexane; des hydrocarbures aromatiques tels que le benzène, le toluène, les xylènes; des hydrocarbures chlorés tels que le chlorobenzène et les polychlorobenzènes.

Parmi ces solvants le monochlorobenzène et les polychlorobenzènes sont particulièrement intéressants car ils permettent une bonne dissolution des chlorophénols et des acides de Lewis, notamment du trichlorure et du tribromure d'aluminium.

Essentiellement en raison de leurs points d'ébullition on utilise plus préférentiellement encore les dichlorobenzènes et les trichlorobenzènes.

Une variante intéressante du procédé consiste à introduire dans le milieu réactionnel de l'acide iodhydrique, de l'acide bromhydrique ou encore de l'iode ou du brome libres qui, dans le milieu, sont réduits par l'hydrogène en hydracides correspondants.

L'acide iodhydrique (ou l'iode) est utilisé de préférence.

La quantité d'hydracide utilisée n'est pas critique. En général le rapport molaire hydracide (ou

équivalent en halogène correspondant)/polychlorophénol est au moins de $1 \times 10^{-4}$ et de préférence de $1 \times 10^{-2}$. La limite supérieure de ce rapport n'est pas critique. Ce rapport peut atteindre une valeur de 5, mais généralement il n'y a pas lieu qu'il soit supérieur à 2 et de préférence supérieur à 1.

La présence d'acide iodhydrique ou bromhydrique est particulièrement intéressante lorsque l'on opère l'hydrodéchloration des polychlorophénols dans un polychlorobenzène, car elle permet d'éviter l'hydrodéchloration du solvant.

La concentration en polychlorophénol dans le solvant mis en œuvre n'est pas critique.

En raison de la sensibilité à l'eau des acides de Lewis et notamment des halogénures d'aluminium, le milieu réactionnel est de préférence substantiellement anhydre.

Les métaux nobles constituant la base des catalyseurs utilisés dans l'invention sont principalement des métaux du groupe VIII de la classification périodique tels que le ruthénium, le rhodium, le palladium, l'osmium, l'iridium et le platine; le palladium est le métal préféré. Le métal peut être à l'état métallique ou à l'état de composé chimique; généralement on préfère que le métal soit mis en œuvre à l'état métallique car, dans les conditions opératoires, les composés ont tendance à être réduits à l'état métallique. Le catalyseur peut être supporté ou non supporté. Comme support du catalyseur, on peut utiliser tout support inerte connu en soi; comme support convenant plus particulièrement, on peut citer le noir de carbone, la silice, le sulfate de baryum; le noir de carbone est un support préféré. Le catalyseur ainsi que son support sont avantageusement sous forme finement divisée; des surfaces spécifiques supérieures à 100 $m^2/g$ conviennent généralement bien.

La quantité de catalyseur mise en œuvre est telle que la proportion pondérale de métal noble du catalyseur par rapport au composé de formule (I) à traiter est généralement comprise entre 0,01 et 10%, de préférence entre 0,1 et 5%.

La température de la réaction est généralement comprise entre 50 et 350° C et, de préférence entre 100 et 250° C.

La pression partielle d'hydrogène peut varier dans de larges limites et être supérieure, inférieure ou égale à la pression atmosphérique. Plus spécifiquement la pression d'hydrogène est comprise entre 0,1 et 60 bars et, de préférence entre 0,5 et 50 bars. On pourrait avoir recours à des pressions supérieures à 60 bars mais sans que cela se traduise par des avantages particuliers. La pression totale à laquelle s'effectue la réaction dépend essentiellement des conditions de température, de la volatilité de l'acide de Lewis et du solvant mis en œuvre dans ces conditions et de la valeur de la pression partielle d'hydrogène. Il va de soi que la pression totale doit être suffisante pour maintenir le milieu réactionnel liquide.

Comme exemples de polychlorophénols de formule (I) qui peuvent être utilisés comme matières premières dans le procédé conforme à la présente invention, on peut citer:

- le dichloro-2,3 phénol
- le dichloro-2,5 phénol
- le dichloro-3,4 phénol
- le trichloro-2,3,4 phénol
- le trichloro-2,3,6 phénol
- le trichloro-2,4,5 phénol
- le trichloro-2,3,5 phénol
- le trichloro-3,4,5 phénol
- le tétrachloro-2,3,4,6 phénol
- le tétrachloro-2,3,4,5 phénol
- le tétrachloro-2,3,5,6 phénol
- le pentachlorophénol
- le trichloro-2,3,4 méthyl-6 phénol
- le dichloro-2,3 méthyl-6 phénol
- le tétrachloro-2,3,4,6 méthyl-5 phénol
- le dichloro-2,3 méthyl-4 phénol
- le tétrachloro-2,3,5,6 méthyl-4 phénol
- le dichloro-2,5 diméthyl-3,4 phénol
- le dichloro-2,5 éthyl-4 phénol
- le dichloro-2,5 propyl-4 phénol
- le dichloro-2,5 t-butyl-4 phénol
- le trichloro-3,4,6 benzyl-2 phénol
- le dichloro-3,4 méthoxy-2 phénol
- le dichloro-3,6 méthoxy-2 phénol
- le dichloro-4,5 méthoxy-2 phénol
- le dichloro-5,6 méthoxy-2 phénol
- le trichloro-3,4,6 méthoxy-2 phénol
- le trichloro-3,4,5 méthoxy-2 phénol
- le tétrachloro-3,4,5,6 méthoxy-2 phénol
- le dichloro-4,5 méthoxy-3 phénol

- le dichloro-5,6 méthoxy-3 phénol
- le dichloro-2,5 méthoxy-3 phénol
- le trichloro-4,5,6 méthoxy-3 phénol
- le trichloro-2,3,6 méthoxy-3 phénol
- le dichloro-4,5 phénoxy-2 phénol
- le tétrachloro-2,3,5,6 phénoxy-4 phénol
- le dichloro-3,4 éthoxy-2 phénol
- le trichloro-3,4,5 éthoxy-2 phénol
- le dichloro-3,4 phényl-2 phénol
- le trichloro-3,5,6 phényl-2 phénol.

En pratique on fait appel de préférence aux di-, tri-, tétra- et pentachlorophénols non substitués. On peut, sans sortir du cadre de la présente invention, soumettre à l'hydrodéchloration des mélanges de deux ou plus de deux des polychlorophénols précités qui peuvent en outre comprendre des quantités mineures de polychlorophénols ne comportant pas d'atomes de chlore en position méta.

Parmi les phénols comportant un atome de chlore sur l'une au moins des positions en méta par rapport à l'hydroxyle phénolique qui peuvent être préparés par le procédé selon la présente invention, on peut citer:

- le chloro-3 phénol
- le dichloro-3,5 phénol
- le chloro-3 méthyl-6 phénol
- le chloro-3 méthyl-5 phénol
- le chloro-3 méthyl-4 phénol
- le dichloro-3,5 méthyl-4 phénol
- le chloro-5 diméthyl-3,4 phénol
- le dichloro-3,5 éthyl-4 phénol
- le dichloro-3,5 propyl-4 phénol
- le dichloro-3,5 t-butyl-4 phénol
- le chloro-3 benzyl-2 phénol
- le chloro-3 méthoxy-2 phénol
- le chloro-3 méthoxy-6 phénol
- le dichloro-3,5 méthoxy-2 phénol
- le chloro-3 méthoxy-5 phénol
- le chloro-3 phénoxy-6 phénol
- le dichloro-3,5 phénoxy-6 phénol
- le chloro-3 éthoxy-2 phénol
- le chloro-3 phényl-2 phénol.

Le procédé selon l'invention peut être mis en œuvre de façon continue ou discontinue. En fin de réaction le catalyseur est séparé par filtration et peut être recyclé tel quel dans une nouvelle opération d'hydrodéchloration. Les métachlorophénols formés peuvent être séparés du milieu réactionnel par distillation.

Les exemples suivants illustrent l'invention et montrent comment elle peut être mise en pratique.

## Exemple 1

Dans un autoclave de 70 ml en acier inoxydable comportant un revêtement intérieur en tantale, équipé d'un système d'agitation, on charge:

- 1,332 g de pentachlorophénol ($5 \cdot 10^{-3}$ mole)
- 0,4 g d'un catalyseur au palladium déposé sur un charbon actif de surface spécifique 1000 $m^2 \cdot g^{-1}$ contenant 5% en poids de palladium métal (soit 0,02 g de palladium)
- 0,295 g de tribromure d'aluminium ($1,1 \cdot 10^{-3}$ mole)
- 20 ml de cyclohexane

On porte le contenu de l'autoclave à 190°C après avoir fermé ce dernier, puis on introduit de l'hydrogène jusqu'à ce que la pression totale atteigne 50 bars et maintient ces conditions pendant 4 heures.

On refroidit ensuite le contenu de l'autoclave, le dégaze puis le soutire. La masse réactionnelle est ensuite filtrée pour séparer le catalyseur, ce dernier est lavé 3 fois par 20 ml d'éthanol. L'alcool de lavage est ajouté au filtrat et la phase organique est distillée pour éliminer l'alcool et le cyclohexane. On recueille 1 g d'un résidu dans lequel on dose et identifie les chlorophénols par chromatographie en phase vapeur.

Les résultats de l'analyse montrent que la totalité du pentachlorophénol a été transformée [taux de transformation (TT): 100%]. On a identifié dans le résidu de distillation:

- du chloro-3 phénol: rendement par rapport au pentachlorophénol chargé (RR) = 4%
- du dichloro-3,5 phénol: RR = 96%.

Exemple 2

On a reproduit l'exemple 1 mais après avoir remplacé le pentachlorophénol par le tétrachloro-2,3,4,6 phénol. La durée de la réaction est de 6 heures.

Les résultats suivants ont été obtenus:

| | | |
|---|---|---|
| — TT | tétrachlorophénol | 100% |
| — RR | chloro-3 phénol | 96% |
| — RR | phénol | 4% |

Exemple 3

On a reproduit l'exemple 1 après avoir remplacé le tribromure d'aluminium par 0,6 g de chlorure d'aluminium. La température de réaction a été de 180°C et la pression totale après charge de l'hydrogène de 21 bars.

Les résultats obtenus ont été les suivants:

| | | |
|---|---|---|
| — TT | pentachlorophénol | 100% |
| — RR | chloro-3 phénol | 8% |
| — RR | dichloro-3,5 phénol | 92% |

Exemple 4

On reproduit l'exemple 1 en chargeant 4 g (0,015 mole) de pentachlorophénol, 1 g de trichlorure d'aluminium (0,0075 mole), 0,5 g d'un catalyseur au Pd déposé à 5% sur du charbon et 5 ml de benzène.

Après 8 h de réaction à 190°C sous 40 bars de pression totale on obtient un taux de transformation de 100% du pentachlorophénol et des rendements de 92% en dichloro-3,5 phénol, 6% en chloro-3 phénol et 2% en phénol.

Exemple 5

Dans un autoclave en acier inoxydable de 250 ml, équipé d'un système d'agitation, on charge en atmosphère anhydre:

| | | |
|---|---|---|
| — | 10,7 g | de pentachlorophénol pur (0,04 mole) |
| — | 1,35 g | de trichlorure d'aluminium anhydre (0,01 mole) |
| — | 0,4 g | de catalyseur Pd à 5% /charbon |
| — | 15 ml | de trichloro-1,2,4 benzène |
| — | 81 mg | d'acide bromhydrique ($10^{-3}$ mole) |

L'autoclave est purgé de l'air qu'il contient par de l'azote puis de l'hydrogène sec.

On porte la masse réactionnelle à 210°C en alimentant de l'hydrogène de telle sorte que la pression totale dans le réacteur soit voisine de 40 bars.

Après 7 heures de réaction on obtient les résultats suivants (dosages effectués par chromatographie en phase vapeur):

| | | |
|---|---|---|
| — TT | du pentachlorophénol | 100% |
| — RR | en dichloro-3,5 phénol | 95,1% |
| — RR | en chloro-3 phénol | 2,9% |
| — RR | en trichloro-2,3,5 phénol | 2,0% |

Le solvant n'est pas hydrodéchloré.

Exemple 6

On répète l'exemple 5, en remplaçant le pentachlorophénol pur par 10,7 g de pentachlorophénol industriel comprenant:

environ 75% (en poids) de pentachlorophénol (0,0302 mole)
environ 20% (en poids) de tétrachloro-2,3,4,6 phénol (0,0092 mole)
environ 5% de sous-produits de la chloration du phénol.

Après 6 heures de réaction dans les conditions de l'exemple 5, on obtient les résultats suivants:

— TT du pentachlorophénol et du tétrachloro-2,3,4,6 phénol: 100%

Les rendements des différents composés dosés sont calculés en% mole/mole par rapport au pentachlorophénol et au tétrachlorophénol chargés:

| | | |
|---|---|---|
| — RR | en dichloro-3,5 phénol | 52,4% |
| — RR | en chloro-3 phénol | 13,1% |
| — RR | en tétrachloro-2,3,5,6 phénol | 10,3% |
| — RR | en Trichloro-2,3,5 phénol | 8,9% |
| — RR | en trichloro-2,3,4 phénol | 2,9% |
| — RR | en trichloro-2,3,6 phénol | 4,6% |
| — RR | en dichloro-2,5 et dichloro-2,3 phénols | 3,1% |

Le solvant n'est pas pas hydrodéchloré.

Exemple 7

On répète l'exemple 5, en remplaçant l'acide bromhydrique par $10^{-4}$ atome-gramme d'iode et on charge 0,6 g de catalyseur Pd à 5% sur charbon au lieu de 0,4 g.
Après 5 heures de réaction dans les conditions de l'exemple 5, on obtient les résultats suivants:

| | | |
|---|---|---|
| — TT | du pentachlorophénol | 96,6% |
| — RR | en dichloro-3,5 phénol | 67,4% |
| — RR | en tétrachloro-2,3,4,5 et en tétrachloro-2,3,5,6 phénols | 13,4% |
| — RR | en trichloro-2,3,5 phénol | 13,8% |
| — RR | en chloro-3 phénol | 0,3% |

Le solvant n'est pas hydrodéchloré.

Exemple 8

Dans l'appareillage utilisé pour l'exemple 5, on charge en atmosphère anhydre:

| | |
|---|---|
| — 2,66 g | de pentachlorophénol |
| — 0,33 g | de trichlorure d'aluminium |
| — 40 ml | d'orthodichlorobenzène |
| — 9,0 g | d'acide iodhydrique gazeux |
| — 0,2 g | de catalyseur Pd à 5% sur charbon |

On opère comme dans l'exemple 5.
Après 2 heures à 210° C sous une pression totale d'environ 40 bars, on obtient les résultats suivants:

| | | |
|---|---|---|
| — TT | du pentachlorophénol | 86,5% |
| — RR | en dichloro-3,5 phénol | 53,8% |
| — RR | en tétrachloro-2,3,4,5 et en tétrachloro-2,3,5,6 phénols | 16,1% |
| — RR | en trichloro-2,3,5 phénol | 16,6% |

Le solvant n'est pas hydrodéchloré.

**Revendications**

1. Procédé d'obtention sélective de chlorophénols comportant un atome de chlore sur l'une au moins des positions méta par rapport à l'hydroxyle phénolique, par hydrogénation à chaud en phase liquide, en présence d'un catalyseur à base d'un métal noble du groupe VIII de la classification périodique, de polychlorophénols de formule générale (I):

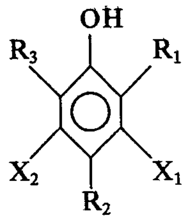

(I)

dans laquelle:

— $X_1$ et $X_2$, identiques ou différents représentent un atome de chlore, un atome d'hydrogène, un radical alkyle, aryle, arylalkyle, alkyloxy, aryloxy, l'un au moins des symboles $X_1$ et $X_2$ représentant un atome de chlore,

— $R_1$, $R_2$ et $R_3$, identiques ou différents, représentent un atome de chlore, un atome d'hydrogène, un radical alkyle, un radical aryle, arylalkyle, un radical alkyloxy, aryloxy, l'un au moins des symboles $R_1$, $R_2$ et $R_3$ représentant un atome de chlore,

caractérisé en ce que la réaction est conduite en phase organique en présence d'au moins un acide de Lewis.

2. Procédé selon la revendication 1, caractérisé en ce que ceux des radicaux $X_1$, $X_2$, $R_1$, $R_2$ et $R_3$ qui ne symbolisent pas un atome de chlore représentent un radical alkyle comportant de 1 à 10 atomes de carbone; un radical phényle; un radical benzyle; un radical alkyloxy comportant de 1 à 10 atomes de carbone; un radical phénoxy.

3. Procédé selon l'une quelconque des revendications 1 à 2, caractérisé en ce que l'acide de Lewis est un halogénure d'éléments des groupes 3a, 4a, 5a, 1b, 2b, 4b, 5b, 6b, 7b et 8 de la classification périodique des éléments.

4. Procédé selon la revendication 3, caractérisé en ce que l'acide de Lewis est un halogénure d'aluminium.

5. Procédé selon la revendication 3, caractérisé en ce que l'halogénure d'aluminium est le chlorure d'aluminium.

6. Procédé selon la revendication 3, caractérisé en ce que l'halogénure d'aluminium est le bromure d'aluminium.

7. Procédé selon l'une quelconque des revendications 1 à 6, caractérisé en ce que la quantité d'acide de Lewis est telle que le rapport molaire acide de Lewis/polychlorophénol de formule (I) est d'au moins $1 \cdot 10^{-4}$.

8. Procédé selon l'une quelconque des revendications 1 à 6, caractérisé en ce que la quantité d'acide de Lewis est telle que le rapport molaire acide de Lewis/polychlorophénol de formule (I) est d'au plus 2.

9. Procédé selon l'une quelconque des revendications 1 à 8, caractérisé en ce que la réaction est conduite en présence d'un solvant organique inerte.

10. Procédé selon la revendication 9, caractérisé en ce que le solvant est le monochlorobenzène ou un polychlorobenzène.

11. Procédé selon la revendication 10, caractérisé en ce que le solvant est un dichlorobenzène ou un trichlorobenzène.

12. Procédé selon la revendication 9, caractérisé en ce que le solvant est un hydrocarbure cycloaliphatique ou aromatique.

13. Procédé selon la revendication 12, caractérisé en ce que le solvant est le cyclohexane ou le benzène.

14. Procédé selon l'une quelconque des revendications 1 à 13, caractérisé en ce que l'on opère en présence d'acide iodhydrique (et/ou d'iode) ou d'acide bromhydrique (et/ou de brome).

15. Procédé selon la revendication 14, caractérisé en ce que l'on utilise un rapport molaire hydracide (ou équivalent en halogéne)/polychlorophénol compris entre $1 \times 10^{-4}$ et 5 et de préférence compris entre $1 \times 10^{-2}$ et 1.

16. Procédé selon l'une quelconque des revendications 1 à 15, caractérisé en ce que le catalyseur est du palladium déposé sur un support inerte.

17. Procédé selon l'une quelconque des revendications 1 à 16, caractérisé en ce que la quantité de catalyseur exprimée en poids de métal noble pour 100 g de polychlorophénol de formule (I) est comprise entre 0,01 g et 10 g.

18. Procédé selon l'une quelconque des revendications 1 à 17, caractérisé en ce que la température de la réaction est comprise entre 50 et 350° C.

19. Procédé selon l'une quelconque des revendications 1 à 18, caractérisé en ce que la pression partielle d'hydrogène est comprise entre 0,1 et 60 bars.

20. Procédé selon la revendication 1, caractérisé en ce que le polychlorophénol de formule (I) est pris dans le groupe formé par le tétrachloro-2,3,4,6 phénol et le pentachlorophénol.

## Patentansprüche

1. Verfahren zur selektiven Herstellung von Chlorphenolen, die ein Chloratom auf mindestens einer der meta-Stellungen hinsichtlich des phenolischen Hydroxyls enthalten, durch Wärmehydrierung in flüssiger Phase, in Gegenwart eines Katalysators auf der Basis eines Edelmetalls der Gruppe VIII des Perioden-Systems, von Polychlorphenolen mit der allgemeinen Formel (I):

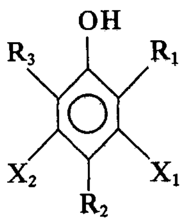

(I)

in der:

— $X_1$ und $X_2$, die identisch oder verschieden sind, ein Chloratom, ein Wasserstoffatom, einen Alkylrest, Aryl, Arylalkyl, Alkyloxy, Aryloxy bedeuten, wobei zumindest eines der Symbole $X_1$ und $X_2$ ein Chloratom darstellt,

— $R_1$, $R_2$ und $R_3$, die identisch oder verschieden sind, ein Chloratom, ein Wasserstoffatom, einen Alkylrest, einen Arylrest, Arylalkyl, einen Alkyloxyrest, Aryloxy bedeuten, wobei zumindest eines der Symbole $R_1$, $R_2$ und $R_3$ ein Chloratom darstellt,

dadurch gekennzeichnet, daß die Reaktion in organischer Phase, in Gegenwart von mindestens einer Lewis-Säure durchgeführt wird.

2. Verfahren gemäß Anspruch 1, dadurch gekennzeichnet, daß diejenigen Reste $X_1$, $X_2$, $R_1$, $R_2$ und $R_3$, die kein Chloratom bedeuten, einen Alkylrest bedeuten, der 1 bis 10 Kohlenstoffatome enthält; einen Phenylrest; einen Benzylrest; einen Alkyloxyrest, der 1 bis 10 Kohlenstoffatome enthält; einen Phenoxyrest.

3. Verfahren gemäß einem der Ansprüche 1 bis 2, dadurch gekennzeichnet, daß die Lewis-Säure ein Halogenid der Elemente der Gruppen 3a, 4a, 5a, 1b, 2b, 4b, 5b, 6b, 7b und 8 des Periodensystems der Elemente ist.

4. Verfahren gemäß Anspruch 3, dadurch gekennzeichnet, daß die Lewis-Säure ein Aluminium-Halogenid ist.

5. Verfahren gemäß Anspruch 3, dadurch gekennzeichnet, daß das Aluminium-Halogenid das Aluminiumchlorid ist.

6. Verfahren gemäß Anspruch 3, dadurch gekennzeichnet, daß das Aluminium-Halogenid das Aluminiumbromid ist.

7. Verfahren gemäß einem der Ansprüche 1 bis 6, dadurch gekennzeichnet, daß die Menge der Lewis-Säure so ist, daß das Molverhältnis Lewis-Säure/Polychlorphenol der Formel (I) mindestens $1 \cdot 10^{-4}$ beträgt.

8. Verfahren gemäß einem der Ansprüche 1 bis 6, dadurch gekennzeichnet, daß die Menge der Lewis-Säure so ist, daß das Molverhältnis Lewis-Säure/Polychlorphenol der Formel (I) höchstens 2 beträgt.

9. Verfahren gemäß einem der Ansprüche 1 bis 8, dadurch gekennzeichnet, daß die Reaktion in Gegenwart eines inerten organischen Lösungsmittels durchgeführt wird.

10. Verfahren gemäß Anspruch 9, dadurch gekennzeichnet, daß das Lösungsmittel das Monochlorbenzol oder ein Polychlorbenzol ist.

11. Verfahren gemäß Anspruch 10, dadurch gekennzeichnet, daß das Lösungsmittel ein Dichlorbenzol oder ein Trichlorbenzol ist.

12. Verfahren gemäß Anspruch 9, dadurch gekennzeichnet, daß das Lösungsmittel ein cycloaliphatischer oder aromatischer Kohlenwasserstoff ist.

13. Verfahren gemäß Anspruch 12, dadurch gekennzeichnet, daß das Lösungsmittel das Cyclohexan oder das Benzol ist.

14. Verfahren gemäß einem der Ansprüche 1 bis 13, dadurch gekennzeichnet, daß in Gegenwart von Jodwasserstoff-Säure oder Bromwasserstoff-Säure gearbeitet wird.

15. Verfahren gemäß Anspruch 14, dadurch gekennzeichnet, daß man ein Molverhältnis Wasserstoffsäure (oder äquivalentes Halogen)/Polychlorphenol zwischen $1 \times 10^{-4}$ und 5 und vorzugsweise zwischen $1 \times 10^{-2}$ und 1 verwendet.

16. Verfahren gemäß einem der Ansprüche 1 bis 15, dadurch gekennzeichnet, daß der Katalysator Palladium ist, das auf einen inerten Träger aufgebracht ist.

17. Verfahren gemäß einem der Ansprüche 1 bis 16, dadurch gekennzeichnet, daß die Katalysatormenge zwischen 0,01 g und 10 g beträgt, ausgedrückt in Edelmetallgewicht für 100 g Polychlorphenol der Formel (I).

18. Verfahren gemäß einem der Ansprüche 1 bis 17, dadurch gekennzeichnet, daß die Reaktionstemperatur zwischen 50 und 350°C beträgt.

19. Verfahren gemäß einem der Ansprüche 1 bis 18, dadurch gekennzeichnet, daß der partielle Druck des Wasserstoffs zwischen 0,1 und 60 bar beträgt.

20. Verfahren gemäß Anspruch 1, dadurch gekennzeichnet, daß das Polychlorphenol der Formel (I) aus der Gruppe genommen wird, die von 2,3,4,6-Tetrachlorphenol und von Pentachlorphenol gebildet wird.

## Claims

1. Process for selectively obtaining chlorophenols containing a chlorine atom in at least one of the metapositions relative to the phenolic hydroxyl, by the hydrogenation, under the action of heat, in the liquid phase, in the presence of a catalyst based on a noble metal of group VIII of the periodic classification, of polychlorophenols of the general formula (I):

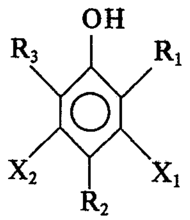

(I)

in which:

- $X_1$ and $X_2$, which are identical or different, represent a chlorine atom, a hydrogen atom or an alkyl, aryl, arylalkyl, alkoxy or aryloxy radical, at least one of the symbols $X_1$ and $X_2$ representing a chlorine atom, and
- $R_1$, $R_2$ and $R_3$, which are identical or different, represent a chlorine atom, a hydrogen atom, an alkyl radical, an aryl or arylalkyl radical or an alkoxy or aryloxy radical, at least one of the symbols $R_1$, $R_2$ and $R_3$ representing a chlorine atom,

characterised in that the reaction is carried out in an organic phase, in the presence of at least one Lewis acid.

2. Process according to Claim 1, characterised in that those radicals $X_1$, $X_2$, $R_1$, $R_2$ and $R_3$ which do not symbolise a chlorine atom represent an alkyl radical containing from 1 to 10 carbon atoms, a phenyl radical, a benzyl radical, an alkoxy radical containing from 1 to 10 carbon atoms or a phenoxy radical.

3. Process according to either one of Claims 1 and 2, characterised in that the Lewis acid is a halide of elements of groups 3a, 4a, 5a, 1b, 2b, 4b, 5b, 6b, 7b and 8 of the periodic classification of the elements.

4. Process according to Claim 3, characterised in that the Lewis acid is an aluminium halide.

5. Process according to Claim 3, characterised in that the aluminium halide is aluminium chloride.

6. Process according to Claim 3, characterised in that the aluminium halide is aluminium bromide.

7. Process according to any one of Claims 1 to 6, characterised in that the amount of Lewis acid is such that the molar ratio Lewis acid/polychlorophenol of the formula (I) is at least $1 \cdot 10^{-4}$.

8. Process according to any one of Claims 1 to 6, characterised in that the amount of Lewis acid is such that the molar ratio Lewis acid/polychlorophenol of the formula (I) is at most 2.

9. Process according to any one of Claims 1 to 8, characterised in that the reaction is carried out in the presence of an inert organic solvent.

10. Process according to Claim 9, characterised in that the solvent is monochlorobenzene or a polychlorobenzene.

11. Process according to Claim 10, characterised in that the solvent is a dichlorobenzene or a trichlorobenzene.

12. Process according to Claim 9, characterised in that the solvent is a cycloaliphatic or aromatic hydrocarbon.

13. Process according to Claim 12, characterised in that the solvent is cyclohexane or benzene.

14. Process according to any one of Claims 1 to 13, characterised in that the reaction is carried out in the presence of hydriodic acid (and/or iodine) or hydrobromic acid (and/or bromine).

15. Process according to Claim 14, characterised in that a molar ratio hydracid (or halogen equivalent)/polychlorophenol of between $1 \times 10^{-4}$ and 5 and preferably of between $1 \times 10^{-2}$ and 1 is used.

16. Process according to any one of Claims 1 to 15, characterised in that the catalyst is palladium deposited on an inert support.

17. Process according to any one of Claims 1 to 16, characterised in that the amount of catalyst, expressed as the weight of noble metal per 100 g of polychlorophenol of the formula (I), is between 0.01 g and 10 g.

18. Process according to any one of Claims 1 to 17, characterised in that the reaction temperature is between 50 and 350° C.

19. Process according to any one of Claims 1 to 18, characterised in that the hydrogen partial pressure is between 0.1 and 60 bars.

20. Process according to Claim 1, characterised in that the polychlorophenol of the formula (I) is taken from the group comprising 2,3,4,6-tetrachlorophenol and pentachlorophenol.